# EUROPÄISCHE PATENTANMELDUNG

(11) **EP 1 862 930 A2**
(43) Veröffentlichungstag der Anmeldung: **05.12.2007**
(21) Anmeldenummer: 07009099.8
(22) Anmeldetag: 05.05.2007
(51) Int. Cl.: G06F 19/00

(54) **Verfahren und Vorrichtung zur automatischen Registrierung eines patientengebundenen medizinischen Gerätes**

(30) Priorität: 30.05.2006 DE 102006024988
(71) Anmelder: BIOTRONIK CRM Patent AG, 6341 Baar (CH)
(72) Erfinder: Urbaszek, Albrecht, Dr., 91353 Hausen (DE); Bode, Sven, Dr., 12203 Berlin (DE); Diebold, Michael, 12169 Berlin (DE); Seidelt, André, 12347 Berlin (DE)
(74) Vertreter: Lindner-Vogt, Karin L.

(57) **Zusammenfassung**

Verfahren zur automatischen Registrierung eines patientengebundenen medizinischen Gerätes bei einem entfernten Datenverarbeitungssystem, wobei Arztidentifikationsdaten an das patientengebundene medizinische Gerät übermittelt werden, die Arztidentifikationsdaten zusammen mit Geräteidentifikationsdaten in einer Registrierungsnachricht zusammengefasst werden, die Registrierungsnachricht vom patientengebundenen medizinischen Gerät an das Datenverarbeitungssystem übermittelt wird und das Datenverarbeitungssystem die in der Registrierungsnachricht enthaltenen Daten prüft und im Erfolgsfalle die Registrierung durchführt, sowie Vorrichtungen hierfür.

## Beschreibung

Die Erfindung betrifft ein Verfahren zur automatischen Registrierung eines patientengebundenen medizinischen Gerätes bei einem entfernten Datenverarbeitungssystem, sowie ein Programmiergerät hierfür nach dem Oberbegriff der Ansprüche 19 und 20, ein patientengebundenes medizinisches Gerät nach dem Oberbegriff des Anspruchs 22 und ein Datenverarbeitungssystem nach dem Oberbegriff des Anspruchs 25.

Zur ständigen Überwachung von Vitalfunktionen des menschlichen Körpers werden Anordnungen verwendet, bei denen ein patientengebundenes medizinisches Gerät die Messung der Körpersignale vornimmt, eine teilweise Erstverarbeitung der Signale durchführt und diese Daten dann an eine entfernte Datenverarbeitungs- und Überwachungszentrale übermittelt. Ein solches System wird in der Offenlegungsschrift WO 03/095024 A2 beschrieben.

Darin wird ferner vorgeschlagen, das patientengebundene medizinische Gerät mit Mitteln auszustatten, vor der Übermittlung konkreter Patientenmessdaten die Bestätigung von zuvor gespeicherten Personen auf elektronischem Wege einzuholen und zu überprüfen, ob ein ebenfalls im Gerät gespeichertes geldwertes elektronisches Guthaben ausreicht, um den Messdatenüberwachungsservice in Anspruch nehmen zu können.

Zur erstmaligen Registrierung eines frisch beim Patienten in Betrieb genommenen patientengebundenen medizinischen Gerätes ist es nach dem Stand der Technik erforderlich, Geräteidentifikationsdaten, beispielsweise die Seriennummer des patientengebundenen Gerätes, vom Arzt im Krankenhaus an eine Benutzerperson am zentralen Überwachungssystem per Telefax oder auf sonstige Weise zu übermitteln und dann die Geräteidentifikationsdaten des patientengebundenen medizinischen Gerätes manuell in eine Eingabemaske des Überwachungssystems einzugeben, um auf diese Weise eine überwachungssystemseitige oder beidseitige Identifikation der Systemkomponenten und/oder Zugriffsautorisierung zu gewährleisten.

Durch diese medienbruchbehaftete, eine manuelle Eingabe erfordernde Übermittlung und Registrierung nach dem Stand der Technik entsteht zum einen der Nachteil, dass zwischen der Initialisierung des patientengebunden medizinischen Gerätes und dessen praktischer Einsatzbereitschaft nach Abschluss des Registrierungsvorganges eine durch den Verwaltungsaufwand bedingte, nachteilige zeitliche Verzögerung entsteht. Die Geräteidentifikationsdaten können zum anderen bei der Übermittlung verfälscht werden oder verloren gehen, was zusätzlichen Aufwand für die Korrektur dieser Fehler erfordert.

Außerdem senkt der erforderliche Aufwand zum Eingeben der Daten die Akzeptanz bekannter Geräte auf Seiten der Ärzte.

Es ist demnach Aufgabe der vorliegenden Erfindung, ein Verfahren und Vorrichtungen anzugeben, welche eine automatische Registrierung eines patientengebundenen medizinischen Gerätes bei einer zentralen Datenüberwachungsstation ermöglicht und die im Stand der Technik genannten Nachteile vermeidet.

Diese Aufgabe wird erfindungsgemäß durch den Gegenstand der Ansprüche 1, 19 und 20, 22, 25 und 28 gelöst.

Die erfindungsgemäße Lösung nach Anspruch 1 wird dargestellt durch ein Verfahren zur automatischen Registrierung eines patientengebundenen medizinischen Gerätes bei einem entfernten Datenverarbeitungssystem, wobei:
- Arztidentifikationsdaten an das patientengebundene medizinische Gerät übermittelt werden,
- die Arztidentifikationsdaten zusammen mit Geräteidentifikationsdaten in einer Registrierungsnachricht zusammengefasst werden,
- die Registrierungsnachricht vom patientengebundenen medizinischen Gerät an das Datenverarbeitungssystem übermittelt wird und
- das Datenverarbeitungssystem die in der Registrierungsnachricht enthaltenen Daten prüft und im Erfolgsfalle die Registrierung durchführt.

Sie weist dabei die folgenden Vorteilhaftigkeiten auf:

Indem die Arztidentifikationsdaten zusammen mit Geräteidentifikationsdaten in einer Registrierungsnachricht zusammengefasst vom patientengebundenen medizinischen Gerät an das Datenverarbeitungssystem übermittelt werden, wird ohne Zwischenschaltung potentiell fehlerbehafteter Tätigkeit durch einen menschlichen Bediener und auf schnelle und vorhandene Infrastruktureinrichtungen einbeziehende Weise der für den Registrierungsvorgang an der zentralen Datenverarbeitungs-/Überwachungsstation erforderliche Datensatz manipulationssicher zur Verfügung gestellt. Auf diese Weise wird es ermöglicht, bei allen auf den Registrierungsvorgang folgenden Ein- und Ausbuchungsvorgängen die Identität des patientengebundenen medizinischen Gerätes durch die zentrale Datenverarbeitungs-/Überwachungsstation automatisch zu überprüfen.

Dadurch, dass in der Registrierungsnachricht die Arztidentifikations- und Geräteidentifikationsdaten in geschlossener Form zusammengefasst sind, wird einerseits die eindeutige Zuordnung der Identifikationsdaten zueinander sichergestellt. Andererseits werden die Daten auf diese Weise in eine Form eines Datencontainers gebracht, welcher durch das Datenverarbeitungssystem unmittelbar als Registrierungsnachricht zu erkennen ist und die Registrierung des patientengebundenen medizinischen Gerätes im Datenverarbeitungssystem auslöst, nachdem sie auf elektronischem Wege übermittelt worden ist.

Dadurch, dass der Vorgang der Prüfung der in der empfangenen Registrierungsnachricht enthaltenen Daten im Datenverarbeitungssystem als zentraler Überwachungsstation stattfindet, können ggf. für die Prüfungsdurchführung wichtige Parameterdaten im System kurzfristig geändert werden und es wird durch die zentrale Speicherung aller relevanten Validierungs- und Authentifizierungsinformationen die Konsistenthaltung dieser für die Registrierung entscheidungsrelevanten Daten erheblich erleichtert. Ferner kann durch die verbesserten Möglichkeiten einer Abschirmung des Zentralsystems unerwünschter Manipulationszugriff auf die Validierungs- und Authentifizierungsdaten technisch aufwandsärmer vermieden werden.

Die erfindungsgemäße Lösung nach Anspruch 19 wird dargestellt durch ein Programmiergerät zur Programmierung eines patientengebundenen medizinischen Gerätes mit mindestens einer Schnittstelle zum Senden und Empfangen von Daten und mit einem Speicher zum Ablegen und Abrufen von Daten, Mitteln zur Eingabe von Benutzerdaten und Mitteln zur Abfrage von Geräteidentifikationsdaten vom patientengebundenen medizinischen Gerät, dadurch gekennzeichnet, dass das Programmiergerät ausgebildet ist zur Erzeugung einer Datenstruktur aus den Geräteidentifikationsdaten und den Benutzerdaten und ferner ausgebildet ist zur Serialisierung und Übermittlung der Datenstruktur als Registrierungsnachricht an das patientengebundene medizinische Gerät.

Indem das Programmiergerät nach Anspruch 19 ausgebildet ist zur Erzeugung einer Datenstruktur aus den Geräteidentifikationsdaten und den Benutzerdaten wird es ermöglicht, den Datenverarbeitungsschritt der Zusammenstellung der registrierungsrelevanten Daten in der Systemkomponente Programmiergerät durchzuführen, die typischerweise im Gegensatz zu den Systemkomponenten des patientengebundenen medizinischen Gerätes einfacher mit Speicher-, Prozessor- und Energieressourcen versorgt werden kann. Ferner können hierdurch an dieser Stelle Benutzerdaten, unter Hinzufügung von personenbezogenen Arzt- oder Patientendaten, mit den Geräteidentifikationsdaten zusammengefasst werden und die im Programmiergerät fertig gestellte Registrierungsnachricht an das patientengebundene medizinische Gerät zur Weiterübermittlung an das entfernte Datenverarbeitungssystem übermittelt werden.

Sowohl in diesem Zusammenhang wie auch im gesamten übrigen Rahmen des in dieser Anmeldung vorgeschlagenen Gegenstandes kann die Ausbildung von Vorrichtungsmerkmalen beliebig durch mehr oder weniger spezifische Hardware und Hardwarekonfigurationen wie auch durch programmtechnische Einrichtung und deren Zusammenwirken erfolgen.

Die erfindungsgemäße Lösung nach Anspruch 20 wird dargestellt durch ein Programmiergerät zur Programmierung eines patientengebundenen medizinischen Gerätes mit mindestens einer Schnittstelle zum Senden und Empfangen von Daten und mit einem Speicher zum Ablegen und Abrufen von Daten, Mitteln zur Eingabe von Benutzerdaten und Mitteln zur Abfrage von Geräteidentifikationsdaten vom patientengebundenen medizinischen Gerät, dadurch gekennzeichnet, dass das Programmiergerät ausgebildet ist zur Erzeugung einer Datenstruktur aus den Geräteidentifikationsdaten und den Benutzerdaten, ausgebildet ist zur Serialisierung und Übermittlung der Datenstruktur als Registrierungsanforderung an ein Datenverarbeitungssystem und ferner ausgebildet ist zur Erzeugung und Übermittlung eines Auslösebefehls für eine Registrierungsnachricht an ein patientengebundenes medizinisches Gerät.

Dadurch, dass das Programmiergerät nach Anspruch 20 ausgebildet ist, um einerseits einen Auslösebefehl für eine Registrierungsnachricht an ein patientengebundenes medizinisches Gerät zu übermitteln und andererseits aus Benutzerdaten, welche personenbezogene Arzt- oder Patientendaten im Klartext enthalten können, zusammen mit Geräteidentifikationsdaten unmittelbar an das entfernte Datenverarbeitungssystem als zentrale Überwachungsstation zu senden, wird ermöglicht, ein erstes, datenmäßig leichtgewichtiges Datenpaket als Registrierungsnachricht auf einen ersten Kommunikationsweg und ein zweites, eine größere Datenmenge umfassendes Datenpaket als Registrierungsanforderung auf einem zweiten Datenweg zu versenden.

Auf diese Weise wird vorteilhafter Weise erstens die Zuverlässigkeit der automatisierten Registrierung erhöht und zweitens die Menge der zu übertragenden Daten an die Kapazität des jeweiligen, die Daten übertragenden Datenkanals angepasst.

Beiden Erfindungen nach den Ansprüchen 19 und 20 ist der Vorteil gemeinsam, dass ein der Kapazität und den besonderen Anforderungen an Zuverlässigkeit und Datensicherheit des jeweiligen Kommunikationskanals angepasster Datencontainer (Registrierungsnachricht, Registrierungsanforderung) erzeugt und mit Daten gefüllt wird und dadurch der Datencontainer automatisiert erkennbar und sein Inhalt automatisiert verarbeitbar ist.

Die erfindungsgemäße Lösung nach Anspruch 22 wird dargestellt durch ein patientengebundenes medizinisches Gerät mit mindestens einer Kommunikationsschnittstelle und gespeicherten Geräteidentifikationsdaten, dadurch gekennzeichnet, dass es ausgebildet ist zur Übertragung einer Registrierungsnachricht an ein Datenverarbeitungssystem. Typische Bestandteile eines derartigen patientengebundenen Gerätes sind ein Implantat, zum Beispiel ein Herzschrittmacher oder Defibrillator, und ein Patientengerät, das von einem Patienten mitgeführt wird und das drahtlos mit dem Implantat kommuniziert. Das Implantat arbeitet grundsätzlich auch unabhängig vom Patientengerät. Für die hier betroffene Erfindung kommt es jedoch gerade auf das Zusammenwirken von Patientengerät und Implantat an, so dass beide Geräte zusammen als patientengebundenes Gerät bezeichnet werden.

Diese Lösung weist dadurch, dass das Gerät ausgebildet ist zur Übertragung einer Registrierungsnachricht an ein Datenverarbeitungssystem, die vorteilhafte Wirkung auf, dass das patientengebundene medizinische Gerät neben der ihm eigenen Funktion der Messung und Übertragung von Messdaten somit ferner eingerichtet ist, eine als solche eindeutig erkennbare Registrierungsnachricht mit den für die Registrierung relevanten Daten an ein Datenverarbeitungssystem zu übermitteln, welches die Registrierungsnachricht als solche erkennt und die Verarbeitung der in der Registrierungsnachricht enthaltenen Daten vornimmt.

Die erfindungsgemäße Lösung nach Anspruch 25 wird dargestellt durch ein Datenverarbeitungssystem mit mindestens einer Kommunikationsschnittstelle zum Empfangen und Versenden von Nachrichten und einem Speicher zum Ablegen und Abrufen von Daten, dadurch gekennzeichnet, dass es ausgebildet ist zum Empfang einer Registrierungsnachricht von einem patientengebundenen medizinischen Gerät, zur Überprüfung der darin enthaltenen Daten, und zur Registrierung und zur Übermittlung einer Bestätigungsnachricht oder einer Fehlernachricht an ein patientengebundenes medizinisches Gerät.

Das so ausgebildete Datenverarbeitungssystem weist dadurch den Vorteil auf, dass es unter Verwendung von im Datenverarbeitungssystem angeordneten System- und Kommunikationseinrichtungen eine Registrierungsnachricht von einem patientengebundenen Gerät empfangen und erkennen kann, und prüfen kann, ob die Daten korrekt und vollständig empfangen worden sind und ob die Registrierung insgesamt zulässig ist, ggf. die in der Registrierungsnachricht enthaltenen Daten für die Registrierung verwenden kann und in jedem Falle eine Nachricht über den Erfolg der automatischen Registrierung an das patientengebundene medizinisches Gerät und das Programmiergerät zurück melden kann.
Die erfindungsgemäße Lösung nach Anspruch 28 wird dargestellt durch eine Anordnung zur Ausführung des Verfahrens zur automatischen Registrierung eines patientengebundenen medizinischen Gerätes bei einem entfernten Datenverarbeitungssystem nach einem der Ansprüche 1 bis 18 mit einem Programmiergerät nach einem der Ansprüche 19 bis 21, einem patientengebundenen medizinischen Gerät nach einem der Ansprüchen 22 bis 24 und einem Datenverarbeitungssystem nach einem der Ansprüche 25 bis 27.

Vorteilhafte Weiterbildungen der Erfindungen nach den Ansprüchen 1, 19, 20, 22 bzw. 25 sind gemäß der jeweils darauf rückbezogenen Unteransprüche möglich und werden im Folgenden kurz erläutert:

Das Verfahren nach Anspruch 1 kann vorteilhafter Weise dahingehend weitergebildet werden, dass die Geräteidentifikationsdaten aus Identifikationsdaten einzelner Systemkomponenten des patientengebunden medizinischen Gerätes zusammengesetzt werden. Auf diese Weise wird es im Zuge der automatischen Registrierung ermöglicht, die einzelnen Systemkomponenten, aus denen sich das patientengebundene medizinische Gerät zusammen setzen kann, wie beispielsweise Implantat einerseits und Patientengerät andererseits, eindeutig zu identifizieren und etwa eine Kompatibilitätsprüfung der Systemkomponenten vorzunehmen.

Eine solche Kompatibilitätsprüfung ist nach einer vorteilhaften Weiterbildung des Verfahrens möglich und kann durch das Datenverarbeitungssystem vorgenommen werden. Ein weiteres vorteilhaftes Ausführungsmerkmal sieht dabei vor, bei der Prüfung der in der Registrierungsnachricht enthaltenen Daten durch das Datenverarbeitungssystem als zentrale Überwachungseinrichtung die Autorisierung des Registrierungsvorganges anhand der in der Registrierungsnachricht enthaltenen Arztidentifikationsdaten zu überprüfen.

Nach einer Ausführungsvariante des Verfahrens werden die Geräteidentifikationsdaten von einem Programmiergerät aus dem patientengebundenen medizinischen Gerät zunächst abgefragt und mit im Programmiergerät gespeicherten Arztidentifikationsdaten zur Registrierungsnachricht zusammengefasst und vom Programmiergerät an das patientengebundene medizinische Gerät übermittelt. Hierdurch ist es nicht nur möglich, die Registrierungsnachricht über die systemimmanenten Kommunikationswege zu übermitteln, sondern es werden auch die Erstellungsvorgänge der Registrierungsnachricht im Programmiergerät, welchem typischerweise leistungsfähigere Systemressourcen zur Verfügung gestellt werden können, und welches typischerweise eine bessere Benutzerschnittstelle enthält als ein patientengebundenes medizinisches Gerät, durchgeführt. Eine vorteilhafte Ausführungsform dieser Variante sieht vor, dass in die Arztidentifikationsdaten weitere personenbezogene Daten, etwa über Arzt und Patient, oder weitere Verwaltungsdaten eingefügt werden. Weiterhin sieht hier eine vorteilhafte Ausführungsform vor, dass das Datenverarbeitungssystem im Falle erfolgreicher Registrierung eine Bestätigung und im Falle fehlgeschlagener Registrierung eine Fehlermeldung an das patientengebundene medizinische Gerät übermittelt, wodurch die systemeigenen Kommunikationsressourcen hier in bidirektionaler Weise auch für die Rückmeldung über den Registrierungsprozess genutzt werden.

Eine weitere Ausführungsvariante des Verfahrens sieht vor, dass vom Programmiergerät an das patientengebundene medizinische Gerät ein Arztidentifikationsdatum übermittelt wird, für welches vorteilhafter Weise ein Schlüsselausdruck verwendet wird. Auf diese Weise wird das Volumen der durch das patientengebundene medizinische Gerät zu übermittelnden Daten und der im patientengebundenen medizinischen Gerät benötigte Speicherplatz verringert. Dieser Vorteil tritt besonders zu Tage, wenn das Verfahren dahingehend ausgebildet ist, dass vom Programmiergerät ein Befehl zur Auslösung einer Registrierungsnachricht an das patientengebundene medizinische Gerät übermittelt wird und im patientengebundenen medizinischen Gerät das Arztidentifikationsdatum mit den im patientengebundenen medizinischen Gerät gespeicherten Geräteidentifikationsdaten zur Registrierungsnachricht zusammengefasst wird. Auf diese Weise wird ein datenmäßig leichtgewichtiges Datenpaket dort erzeugt, wo die systemkritischen Geräteidentifikationsdaten bereits fest abgelegt sind. Auf diese Weise werden Übermittlungsfehler vermindert und manuelle Eingaben oder Übertragungen von Geräteidentifikationsdaten vermieden.

Eine vorteilhafte Ausführungsform dieser Variante sieht vor, dass vom Programmiergerät eine Registrierungsanforderung an das Datenverarbeitungssystem übermittelt wird. Auf diese Weise kann über einen zweiten, vom patientengebundenen medizinischen Gerät unabhängigen Kommunikationsweg (z. B. Internet) eine parallele und mit zusätzlichen Daten versehene Registrierungsanforderung versendet werden, in welche vorteilhafter Weise Geräteidentifikationsdaten und/oder Arztidentifikationsdaten und/oder personenbezogene Daten eingefügt werden können. Das Verfahren kann vorteilhafter Weise dahingehend fortgebildet werden, dass das Datenverarbeitungssystem im Falle erfolgreicher Registrierung eine Bestätigung an das Programmiergerät unmittelbar übermittelt und anderenfalls eine Fehlermeldung.

Wird das Verfahren vorteilhafter Weise dahingehend weitergebildet, dass im Datenverarbeitungssystem die in der Registrierungsnachricht enthaltenen Daten mit den in der Registrierungsanforderung enthaltenen Daten abgeglichen werden, so wird bei der automatischen Erkennung und Verarbeitung der Registrierungsnachricht und der Registrierungsanforderung die Gültigkeit der enthaltenen Daten und/oder die Zulässigkeit der Registrierung nicht nur durch einen Abgleich mit den im Datenverarbeitungssystem zentral gespeicherten Daten festgestellt, sondern zudem die Konsistenz der Daten durch die Zusammenführung von Registrierungsnachricht und -anforderung sichergestellt.

Bei der Überprüfung im Datenverarbeitungssystem ist es vorteilhaft, im Rahmen einer vorteilhaften Weiterbildung des Verfahrens zu prüfen, ob für eine empfangene Registrierungsanforderung genau eine Registrierungsnachricht empfangen worden ist (oder umgekehrt für eine empfangene Registrierungsnachricht genau eine empfangene Registrierungsanforderung). Für die vorteilhafte Auswahl von Kommunikationskanälen im Rahmen des Verfahrens kann erstens zwischen dem Programmiergerät und dem patientengebunden medizinischen Gerät eine Infrarotverbindung (vorteilhafter Weise in Form einer seriellen IR-Schnittstelle), zweitens zwischen dem Programmiergerät und dem Datenverarbeitungssystem eine Internetverbindung und drittens zwischen dem patientengebundenen medizinischen Gerät und dem Datenverarbeitungssystem eine Mobilfunkverbindung hergestellt werden.

Um die Identität und die Kompatibilität einzelner Systemkomponenten des patientengebundenen medizinischen Gerätes festzustellen, ist es vorteilhaft, das Programmiergerät dahingehend weiterzubilden, dass die darin zusammengesetzte Datenstruktur ein erstes Datenfeld zur Speicherung des Geräteidentifikationsdatums einer ersten Systemkomponente des patientengebundenen medizinischen Gerätes aufweist sowie ein zweites Datenfeld zur Speicherung des Geräteidentifikationsdatums einer zweiten Systemkomponente des patientengebundenen medizinischen Gerätes und weitere Datenfelder zur Speicherung von Benutzerdaten, welche personenbezogene Daten über Arzt und/oder Patient enthalten können, sowie Identifikationsdaten, die zur Kennzeichnung als Registrierungsnachricht dienen.

Wird das patientengebundene medizinische Gerät vorteilhafter Weise derart weitergebildet, dass es ausgebildet ist zum Empfang und zur Verarbeitung von Arztidentifikationsdaten, sowie zum Empfang und zur Verarbeitung eines Auslösebefehls für eine Registrierungsnachricht vom Programmiergerät, so wird ermöglicht, in unmittelbar unabhängiger Weise ein Arztidentifikationsdatum und separat einen Befehl zur Erstellung einer Registrierungsnachricht entgegen zu nehmen. Auf Grund des Auslösebefehls für eine Registrierungsnachricht wird gemäß einer Ausführungsform des patientengebundenen medizinischen Gerätes durch eine dementsprechende programmtechnische Einrichtung eine Datenstruktur aus dem Geräteidentifikationsdaten und dem zuvor übermittelten Arztidentifikationsdatum, welches vorzugsweise eine alphanumerische Zeichenkette sein kann, erzeugt. Weiterhin sind nach der vorteilhaften Weiterbildung programmtechnische Mittel enthalten, welche diese Datenstruktur zu serialisieren, als Registrierungsnachricht zu kennzeichnen und an das Datenverarbeitungssystem zu übermitteln ausgebildet sind.

Die Fortbildungen des erfindungsgemäßen Datenverarbeitungssystems sind den auf Anspruch 25 rückbezogenen Ansprüchen zu entnehmen und weisen den vorstehend erläuterten Verfahrens- und Vorrichtungsfortbildungen entsprechende, vorteilhafte Einrichtungen auf.

Die Erfindung wird anhand zweier Figuren mit Ausführungsbeispielen erläutert. Es zeigen:
- Fig. 1: Systemkomponenten und Kommunikationsvorgänge bei der automatischen Registrierung nach einer ersten Ausführungsvariante, und
- Fig. 2: Systemkomponenten und Kommunikationsvorgänge bei der automatischen Registrierung gemäß einer zweiten Ausführungsvariante.

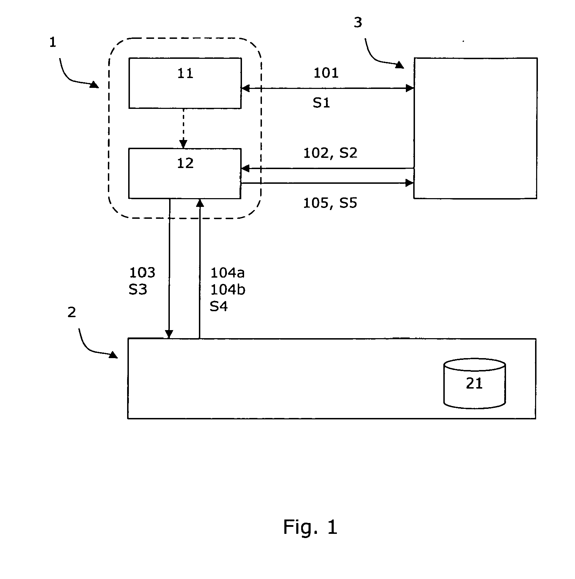

Es zeigt Fig. 1 einen schematischen Überblick über ein Gesamtsystem für das telemedizinische Patientenmonitoring mit den Systemkomponenten patientengebundenes medizinisches Gerät 1, Datenverarbeitungssystem als zentrale Überwachungsstation 2 und Programmiergerät 3. Das patientengebundene medizinische Gerät 1 befindet sich ständig in unmittelbarer Nähe des zu überwachenden Patienten und besteht im vorliegenden Falle aus zwei Systemkomponenten, Implantat 11 und Patientengerät 12, welche über eine drahtlose Kommunikationsschnittstelle verbunden sind, die unidirektional vom Implantat zum Patientengerät oder bidirektional gestaltet sein kann. Zur Initialisierung und/oder Programmierung wird typischerweise die Programmiereinrichtung 3 in die räumliche Nähe zum patientengebundenen Gerät 1 gebracht. Von diesen vorgenannten Komponenten deutlich entfernt angeordnet, ist das Datenverarbeitungssystem 2 als zentrale Überwachungseinrichtung für typischerweise eine Vielzahl von patientengebundenen Geräten 1.

In diesem Beispiel findet daher die Kommunikation zwischen patientengebudenem Gerät 1, genauer: zwischen Patientengerät 12, und Datenverarbeitungssystem 2 über eine Mobilfunkverbindung statt, während die Kommunikation zwischen patientengebundenem Gerät 1 und Programmiergerät 3 über eine Infrarotschnittstelle zwischen Patientengerät 12 und Programmiergerät 3 und/oder eine weitere drahtlose Lokalverbindung zwischen Implantat 11 und Programmiergerät 3 erfolgt.

Das Datenverarbeitungssystem 2 weist eine Datenbank 21 auf, in der eine Vielzahl verschiedener Daten gespeichert ist, so etwa technische Daten zu Typen und einzelnen Exemplaren von patientengebundenen medizinischen Geräten, personenbezogene Daten über Arzt und Patient, die überwachten Messdaten der Vitalfunktionen des Patienten oder Software-Komponenten für die Fernwartung des patientengebunden medizinischen Gerätes.

Im Rahmen dieser gesamten Patentanmeldung und sämtlicher Ausführungsbeispiele bezeichnet "personenbezogene Daten" typischerweise in mehreren Feldern angeordnete alphanumerische Zeichenketten.

Bevor im laufenden Betrieb das patientengebundene medizinische Gerät 1 über das Patientengerät 12 in periodischen Abständen Messdaten an das entfernte Datenverarbeitungssystem 2 über eine Mobilfunkverbindung übermittelt, muss, möglichst zeitnah nach der Erstinbetriebnahme von Implantat 11 und Patientengerät 12, eine Registrierung des patientengebundenen medizinischen Gerätes 1 beim Zentralsystem 2 stattfinden.

Hierzu wird zunächst das Implantat im bidirektionalen Kommunikationsvorgang S1 vom Programmiergerät 3 abgefragt. Im vorliegenden Falle wird dies durch eine lokale Funkverbindung realisiert, es ist alternativ aber auch die Kommunikation über Infrarotschnittstelle mit dem Patientengerät 12 denkbar, welches die Informationsanforderung entsprechend über eine lokale Funkverbindung an das Implantat 11 weiterleitet und entsprechend die vom Implantat 11 empfangene, abgefragte Information über die Infrarotschnittstelle an das Programmiergerät 3 übermittelt.

Nach Abfrage des Implantats 11 (in diesem Falle unmittelbar über eine eigene lokale Funkverbindung) wird eine Infrarotverbindung zwischen dem Programmiergerät 3 und dem Patientengerät 12 in nicht näher dargestellter Weise hergestellt. Dann werden im Programmiergerät 3 die Benutzerdaten, welche neben den personenbezogenen Daten des behandelnden Arztes auch weitere Daten über den Patienten sowie weitere Nutzdaten umfassen, durch den Arzt kontrolliert und durch Angabe seiner Nutzeridentifikation bestätigt, wobei sich der Arzt durch Angabe von Nutzergruppe, Name und Passwort authentifizieren muss.

Darauf werden die aus dem Implantat 11 in Schritt S1 abgefragten Geräteidentifikationsdaten 101 im Programmiergerät 3 mit den Benutzerdaten, welche die genannten Arztidentifikationsdaten und übrigen Daten enthalten, in einer als Registrierungsnachricht gekennzeichneten Datenstruktur 102 zusammengefasst, welche serialisiert an das Patientengerät 12 im Kommunikationsschritt S2 zur Weiterübertragung an das entfernte Datenverarbeitungssystem 2 übermittelt wird. Die Weiterreichung der im Programmiergerät 3 zusammengesetzten Registrierungsnachricht 102 durch das Patientengerät 12 erfolgt in Schritt S3 über eine Mobilfunkverbindung (GPRS). Das Datenverarbeitungssystem 2 verarbeitet die empfangene Registrierungsnachricht 103, indem sie die aus der Seriennummer des Implantats 11 und der Seriennummer des Patientengerätes 12 bestehenden Geräteidentifikationsdaten und die Arztidentifikationsdaten und weiteren Benutzerdaten ausliest und prüft, ob der Benutzer zur Registrierung des patientengebundenen Gerätes 1 befugt ist und ob Implantat und Patientengerät zueinander kompatibel sind. Hierzu greift das Datenverarbeitungssystem 2 auf in Datenbank 21 gespeicherte Daten zurück. Ist das Ergebnis der automatisierten Prüfung, in die weitere technische und administrative Informationen und Rahmenbedingungen einbezogen werden können, positiv, so wird in Kommunikationsschritt S4 eine Erfolgsmeldung 104a übermittelt (im Falle eines negativen Prüfungsergebnisses eine Fehlermeldung 104b) welche vom Patientengerät 12 empfangen und über Infrarotschnittstelle in Kommunikationsschritt S5 an das Programmiergerät 3 weitergeleitet wird. Das Programmiergerät 3 gibt eine entsprechende Bestätigungs- oder Fehlermeldung am Bildschirm aus.

In analoger Weise kann die Deregistrierung, also die endgültige Abmeldung aus der laufenden Messdatenüberwachung, vorgenommen werden.

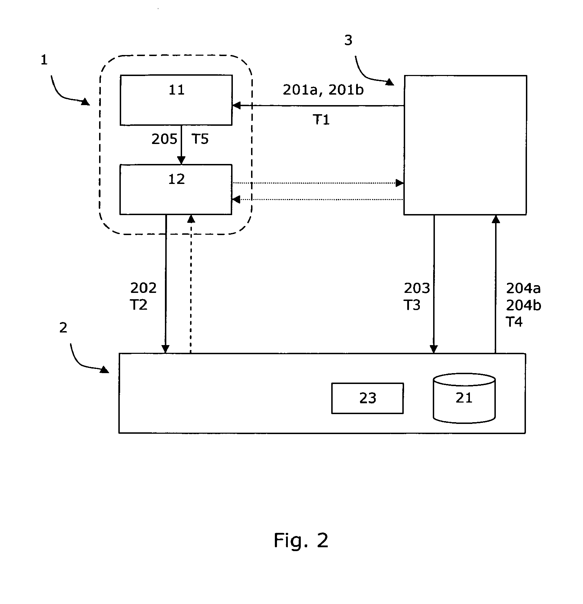

Fig. 2 zeigt eine alternative Ausführung für den Verfahrens- und Kommunikationsablauf der automatischen Registrierung. Die grundsätzliche Funktionalität der jeweiligen Systemkomponenten, patientengebundenes medizinisches Gerät 1, entferntes Datenverarbeitungssystem 2 und Programmiergerät 3, sind im Wesentlichen gleich, auch und insbesondere hinsichtlich des laufenden Betriebes bei der Übermittlung von Signaldaten des überwachten Patienten, aber auch für Holter- und Statistikspeicherdaten mit Informationen bspw. über den Verlauf der Therapie zwischen Nachsorgevorgängen, aufgetretene Episoden oder technische Funktionsparameter, sowie nachfolgend beschriebene Bedienprozeduren und -elemente.

Wird in dieser Ausführungsvariante das Verfahren zur automatischen Registrierung durch den Arzt am Programmiergerät 3, durch Betätigung eines Buttons oder einer Checkbox ausgelöst, wobei ein solches Bedienerelement zur optimalen Unterstützung des Arztes etwa nur dann erscheinen sollte, wenn das Implantat noch nicht registriert wurde, so wird im Gegensatz zur in Fig.1 dargestellten Ausführungsvariante hier zunächst in Kommunikationsschritt T1 ein Arztidentifikationsdatum 201a in Form eines alphanumerischen Arztschlüssels und eines Registrierungsbefehles 201b an das Implantat 11 übermittelt. Die Übermittlung von Arztidentifikationsdatum 201a und Registrierungsbefehl 201b kann dabei zeitlich unabhängig voneinander erfolgen, oder es kann das Arztidentifikationsdatum zusammen mit dem Registrierungsbefehl im Rahmen einer Programmübertragung des Programmiergerätes 3 an das Implantat 11 erfolgen, so dass das Implantat 11 veranlasst wird, unmittelbar nach Beendigung des Programmiervorganges in Kommunikationsschritt T1 eine Registrierungsnachricht 205 zu erzeugen und versenden. Nach Absenden des Registrierungsbefehls 201b im Rahmen des Kommunikationsschrittes T1 zeigt das Programmiergerät 3 eine Hinweismeldung am Bildschirm an, dass das Patientengerät 12 einzuschalten ist und sicher zu stellen ist, dass kein weiteres Patientengerät in Sendereichweite des Implantats 11 befindlich ist.

In nicht näher dargestellter Weise wird der vom Implantat 11 empfangene Registrierungsbefehl 201b ausgeführt, indem das Arztidentifikationsdatum mit den im Implantat 11 gespeicherten Geräteidentifikationsdaten (der Seriennummer des Implantats) und der Seriennummer des Patientengerätes 12, welches zuvor aus dem selben über eine bidirektionale, lokale Funkverbindung abgefragt wurde, in einer Datenstruktur resp. einem Datencontainer zusammengefasst.

Hierbei kann der Datencontainer in eine spezifische Form gebracht werden, so dass er eindeutig als Registrierungsnachricht erkannt werden kann. Hierzu bietet sich das Voranstellen eines Headers mit einer zugewiesenen Kennung an.

Dann wird die so entstandene Registrierungsnachricht 205 im Kommunikationsschritt T5 an das Patientengerät 12 übermittelt. Das Patientengerät wiederum übermittelt die Registrierungsnachricht über eine Mobilfunkverbindung im Kommunikationsschritt T2 an das Datenverarbeitungssystem 2.

Alternativ ist möglich, dass im Implantat 11 zunächst eine Registrierungsnachricht 205 erzeugt wird, welche zunächst lediglich das Geräteidentifikationsdatum des Implantats 11 aufweist und das Geräteidentifikationsdatum des Patientengerätes 12 erst nach dem Kommunikationsschritt T5 im Patientengerät 12 eingefügt wird, bevor die Registrierungsnachricht 202 im Kommunikationsschritt T2 an die Datenverarbeitungszentrale weitergeleitet wird. Diese Variante kann auch dann realisiert werden, wenn die Verbindung zwischen Implantat 11 und Patientengerät eine unidirektionale Verbindung vom Implantat zum Patientengerät ist.

Gleichzeitig oder nach der Absendung bzw. Programmübertragung im Kommunikationsschritt T1 wird im Programmiergerät 3 eine Registrierungsanforderung 203 erzeugt, welche den gleichen alphanumerischen Kurzschlüssel 201a als Arztidentifikationsdatum enthält, wie er zuvor im Schritt T1 an das Implantat 11 übermittelt wurde. Diese Registrierungsanforderung 203 enthält weiterhin personenbezogene Angaben zum Arzt und zum Patienten, sowie die Geräteidentifikationsdaten des patientengebundenen medizinischen Gerätes 1 (mindestens die des Implantats 11) sowie weitere Benutzerdaten mit technischem und/oder administrativem Verwendungszweck. Als Registrierungsanforderung in einen Datencontainer zusammengefasst und als solche in ähnlicher Weise wie bei der Registrierungsnachricht kenntlich gemacht wird sie im Kommunikationsschritt T3, parallel zur über die Mobilfunkverbindung übertragenen Registrierungsnachricht, über eine leistungsfähige und hinreichend gesicherte Online-Verbindung (z. B. Internet via https) an das Datenverarbeitungssystem 2 übertragen. Nach dem Empfang der Registrierungsanforderung erwartet das Datenverarbeitungssystem 2 die der Registrierungsanforderung entsprechende Registrierungsnachricht innerhalb eines vorbestimmten Zeitfensters, z. B. drei Minuten. Entsprechend kann das Zeitfenster im Falle der zuerst eintreffenden Registrierungsnachricht auch von dieser ausgelöst werden, so dass die zur Registrierungsnachricht passende Registrierungsanforderung innerhalb des Zeitfensters erwartet wird. Das Erkennen einer Registrierungsanforderung oder -nachricht als zur jeweils anderen Nachricht gehörend wird anhand der in den Nachrichten jeweils enthaltenen Geräteidentifikationsdaten vorgenommen.

Treffen innerhalb des vorbestimmten Zeitfensters, dessen Beginn und Dauer der Zeitgeber 23 festlegt, genau eine Registrierungsanforderung und genau eine passende Registrierungsnachricht im Datenverarbeitungssystem 2 ein, so wird das in der Registrierungsnachricht enthaltene Arztidentifikationsdatum und das Geräteidentifikationsdatum auf Übereinstimmung mit der in der zugehörigen Registrierungsanforderung enthaltenen Arztidentifikations- und Geräteidentifikationsdaten überprüft. Stimmen diese Daten überein, so wird nach etwaiger weiterer Überprüfung verschiedener technischer oder administrativer Bedingungen, wie etwa der Abgleich der Seriennummern oder des Arztidentifikationsschlüssels mit in Datenbank 21 enthaltenen Informationen (bspw. Kompatibilitätsinformationen zwischen Implantat 11 und Patientengerät 12, Liste zertifizierter Ärzte), die Registrierung des patientengebundenen medizinischen Gerätes 1 für den Arzt, dessen Arztidentifikationsdatum übertragen wurde, und das Patientengerät, welches die Registrierungsnachricht übertragen hat, vorgenommen. Die im Zuge der Registrierung in der Datenbank 21 zu speichernden Geräteidentifikationsdaten von Implantat 11 und Patientengerät 12 werden aus der Registrierungsnachricht entnommen.

Im Falle der erfolgreichen Registrierung wird in Kommunikationsschritt T4 eine Registrierungsbestätigung 204a über die Online-Verbindung zwischen Programmiergerät 3 und Datenverarbeitungseinrichtung 2 übermittelt und in Programmiereinrichtung 3 wird ein Hinweisfenster für den Benutzer angezeigt, welches folgende Angaben enthält: Implantat-Typ und Geräteidentifikationsdatum (Seriennummer) des unmittelbar zuvor registrierten Implantates, Arztidentifikationsdatum (Arzt-Schlüssel) und Name des Arztes, Typ und Identifikationsdatum des Patientengerätes; auf diese Weise kann der Arzt die Angaben sicherheitshalber erneut überprüfen und den Vorgang seinerseits durch Betätigen eines OK-Buttons als Steuerungselement bestätigen. Treffen innerhalb des angegebenen Zeitfensters jedoch keine oder mehrere Registrierungsnachrichten zu einer Registrierungsanforderung ein, derart, dass die enthaltenen Identifikationsdaten zueinander passen, so wird entsprechend vom Datenverarbeitungssystem 2 in Kommunikationsschritt T4 eine Fehlernachricht 204b an das Programmiergerät 3 übermittelt, worauf im Programmiergerät 3 eine entsprechende Fehlermeldung angezeigt wird, welche die Ursache der fehlgeschlagenen Registrierung, Hinweise zum Abstellen des Problems, sowie die Aufforderung zur Wiederholung der Registrierung enthält.

Für den Deregistrierungsvorgang gemäß dieser Ausführungsvariante wird eine Deregistrierungsanforderung vom Programmiergerät 3 über die Online-Verbindung zum Datenverarbeitungssystem 2 gesendet, wobei diese Deregistrierungsanforderung durch das Betätigen eines Bedienelementes auf der Bedienoberfläche des Programmiergerätes durch den Arzt ausgelöst wird.

## Patentansprüche

1. Verfahren zur automatischen Registrierung eines patientengebundenen medizinischen Gerätes (1) bei einem entfernten Datenverarbeitungssystem (2), wobei
- Arztidentifikationsdaten (102, 201) an das patientengebundene medizinische Gerät (1) übermittelt werden,
- die Arztidentifikationsdaten zusammen mit Geräteidentifikationsdaten in einer Registrierungsnachricht (103, 205, 202) zusammengefasst werden,
- die Registrierungsnachricht (103, 205, 202) vom patientengebundenen medizinischen Gerät (1) an das Datenverarbeitungssystem (2) übermittelt wird und
- das Datenverarbeitungssystem (2) die in der Registrierungsnachricht (103, 202) enthaltenen Daten prüft und im Erfolgsfalle die Registrierung durchführt.

2. Verfahren nach Anspruch 1, **dadurch gekennzeichnet, dass** die Geräteidentifikationsdaten aus Identifikationsdaten einzelner Systemkomponenten (11, 12) des patientengebundenen medizinischen Gerätes (1) zusammengesetzt werden, im Datenverarbeitungssystem (2) die Kompatibilität der Systemkomponenten (11, 12) anhand der Geräteidentifikationsdaten und die Autorisierung anhand der Arztidentifikationsdaten überprüft werden.

3. Verfahren nach einem der Ansprüche 1 oder 2, **dadurch gekennzeichnet, dass** die Geräteidentifikationsdaten (101) von einem Programmiergerät (3) aus dem patientengebundenen medizinischen Gerät (1, 11) abgefragt werden und mit im Programmiergerät (3) gespeicherten Arztidentifikationsdaten, in die personenbezogene Daten eingefügt werden, zur Registrierungsnachricht (102) zusammengefasst vom Programmiergerät (3) an das patientengebundene medizinische Gerät (1, 12) übermittelt werden.

4. Verfahren nach Anspruch 3, **dadurch gekennzeichnet, dass** das Datenverarbeitungssystem (2) im Falle erfolgreicher Registrierung eine Bestätigung (104a) an das patientengebundene medizinische Gerät (1, 12) übermittelt und anderenfalls eine Fehlermeldung (104b).

5. Verfahren nach einem der Ansprüche 1oder 2, **dadurch gekennzeichnet, dass** als Arztidentifikationsdatum ein Schlüsselausdruck (201a) ausgewählt wird, welcher von einem Programmiergerät (3) an das patientengebundene medizinische Gerät (1, 11) übermittelt wird.

6. Verfahren nach Anspruch 5, **dadurch gekennzeichnet, dass** vom Programmiergerät (3) ein Auslösebefehl (201b) zur Auslösung einer Registrierungsnachricht an das patientengebundene medizinische Gerät (1) übermittelt wird und im patientengebundenen medizinischen Gerät (1) das Arztidentifikationsdatum mit den im patientengebundenen medizinischen Gerät gespeicherten Geräteidentifikationsdaten zur Registrierungsnachricht zusammengefasst wird.

7. Verfahren nach einem der Ansprüche 5 oder 6, **dadurch gekennzeichnet, dass** eine Registrierungsanforderung (203) vom Programmiergerät (3) an das Datenverarbeitungssystem (2) übermittelt wird und in die Registrierungsanforderung (203) die Geräteidentifikationsdaten und/oder die Arztidentifikationsdaten und/oder personenbezogene Daten eingefügt werden.

8. Verfahren nach Anspruch 7, **dadurch gekennzeichnet, dass** das Datenverarbeitungssystem (2) im Falle erfolgreicher Registrierung eine Bestätigung (204a) an das Programmiergerät (3) übermittelt und anderenfalls eine Fehlermeldung (204b) übermittelt und dass im Datenverarbeitungssystem (2) die in der Registrierungsnachricht enthaltenen Daten mit den in der Registrierungsanforderung enthaltenen Daten abgeglichen werden und im Datenverarbeitungssystem (2) überprüft wird, ob innerhalb eines vorgegebenen Zeitrahmens Registrierungsnachricht (202) und zugehörige Registrierungsanforderung (203) empfangen werden.

9. Verfahren nach einem der Ansprüche 7 oder 8, **dadurch gekennzeichnet, dass** im Datenverarbeitungssystem (2) geprüft wird, ob für eine empfangene Registrierungsanforderung (203) genau eine Registrierungsnachricht (202) empfangen worden ist.

10. Verfahren nach einem der Ansprüche 3 bis 9, **dadurch gekennzeichnet, dass** zwischen dem Programmiergerät (3) und dem patientengebundenen medizinischen Gerät (1, 12) eine Infrarotverbindung oder eine Internetverbindung oder eine Mobilfunkverbindung hergestellt wird.

11. Programmiergerät (3) zur Programmierung eines patientengebundenen medizinischen Gerätes (1) mit mindestens einer Schnittstelle zum Senden und Empfangen von Daten und mit einem Speicher zum Ablegen und Abrufen von Daten, Mitteln zur Eingabe von Benutzerdaten und Mitteln zur Abfrage von Geräteidentifikationsdaten vom patientengebundenen medizinischen Gerät (1), **dadurch gekennzeichnet, dass** das Programmiergerät ausgebildet ist zur Erzeugung einer Datenstruktur aus den Geräteidentifikationsdaten und den Benutzerdaten und ferner ausgebildet ist zur Serialisierung und Übermittlung der Datenstruktur als Registrierungsnachricht (102) an das patientengebundene medizinische Gerät (1, 12).

12. Programmiergerät (3) zur Programmierung eines patientengebundenen medizinischen Gerätes (1) mit mindestens einer Schnittstelle zum Senden und Empfangen von Daten und mit einem Speicher zum Ablegen und Abrufen von Daten, Mitteln zur Eingabe von Benutzerdaten und Mitteln zur Abfrage von Geräteidentifikationsdaten vom patientengebundenen medizinischen Gerät (1), **dadurch gekennzeichnet, dass** das Programmiergerät ausgebildet ist zur Erzeugung einer Datenstruktur aus den Geräteidentifikationsdaten und den Benutzerdaten, ausgebildet ist zur Serialisierung und Übermittlung der Datenstruktur als Registrierungsanforderung (203) an ein Datenverarbeitungssystem (2) und ferner ausgebildet ist zur Erzeugung und Übermittlung eines Auslösebefehls (201) für eine Registrierungsnachricht an ein patientengebundenes medizinisches Gerät (1, 11).

13. Programmiergerät nach einem der Ansprüche 11 oder 12, **dadurch gekennzeichnet, dass** die Datenstruktur ein erstes Datenfeld zur Speicherung des Geräteidentifikationsdatums einer ersten Systemkomponente (11) eines patientengebundenen medizinischen Gerätes (1) aufweist sowie ein zweites Datenfeld zur Speicherung des Geräteidentifikationsdatums einer zweiten Systemkomponente (12) eines patientengebundenen medizinischen Gerätes (1) und weitere Datenfelder zur Speicherung von Benutzerdaten.

14. Patientengebundenes medizinisches Gerät (1) mit mindestens einer Schnittstelle zum Senden und Empfangen von Daten und mit gespeicherten Geräteidentifikationsdaten, **dadurch gekennzeichnet, dass** es ausgebildet ist zur Übertragung einer Registrierungsnachricht (103, 202) an ein Datenverarbeitungssystem (2).

15. Patientengebundenes medizinisches Gerät (1, 11) nach Anspruch 14, **dadurch gekennzeichnet, dass** es ausgebildet ist zum Empfang und Verarbeitung von Arztidentifikationsdaten (201a), sowie zum Empfang und Verarbeitung eines Auslösebefehls (201b) für eine Registrierungsnachricht von einem Programmiergerät (3).

16. Patientengebundenes medizinisches Gerät nach einem der Ansprüche 14 oder 15, **dadurch gekennzeichnet, dass** es ausgebildet ist zur Erzeugung einer Datenstruktur aus den Geräteidentifikationsdaten und den Arztidentifikationsdaten und ferner ausgebildet ist zur Serialisierung und Übermittlung der Datenstruktur als Registrierungsnachricht (205, 202) an das Datenverarbeitungssystem (2).

17. Datenverarbeitungssystem (2) mit mindestens einer Kommunikationsschnittstelle zum Empfangen und Versenden von Nachrichten (103, 104a, 104b, 202, 203, 204a, 204b) und einem Speicher (21) zum Ablegen und Abrufen von Daten, **dadurch gekennzeichnet, dass** es ausgebildet ist zum Empfang einer Registrierungsnachricht (103, 202) von einem patientengebundenen medizinischen Gerät (1), zur Überprüfung der darin enthaltenen Daten, und zur Registrierung und zur Übermittlung einer Bestätigungsnachricht (104a, 204a) oder Fehlernachricht (104b, 204b) an ein patientengebundenes medizinisches Gerät (1, 12) und/oder an ein Programmiergerät (3).

18. Datenverarbeitungssystem nach Anspruch 17, **dadurch gekennzeichnet, dass** es ausgebildet ist zum Empfang einer Registrierungsanforderung (203) und ferner zur Zusammenführung einer empfangenen Registrierungsanforderung (203) mit einer empfangenen Registrierungsnachricht (202).

19. Datenverarbeitungssystem nach Anspruch 18, **dadurch gekennzeichnet, dass** es einen Zeitgeber (23) aufweist zur Festlegung eines Zeitrahmens und dass es ausgebildet ist zur Überprüfung, ob zu einer Registrierungsanforderung (203) genau eine Registrierungsnachricht (202) innerhalb des Zeitrahmens empfangen wurde.

20. Anordnung zur Ausführung des Verfahrens zur automatischen Registrierung eines patientengebundenen medizinischen Gerätes bei einem entfernten Datenverarbeitungssystem nach einem der Ansprüche 1 bis 10, mit einem Programmiergerät (3) nach einem der Ansprüche 11 bis 13, einem patientengebundenen medizinischen Gerät (1) nach einem der Ansprüche 14 bis 16 und einem Datenverarbeitungssystem (2) nach einem der Ansprüche 17 bis 19.
